# EUROPEAN PATENT APPLICATION

(11) **EP 4 596 556 A1**
(43) Date of publication of application: **06.08.2025**
(21) Application number: 23870771.5
(22) Date of filing: 26.09.2023
(51) Int. Cl.: C07D 495/06, A61K 31/35, A61K 31/381, A61P 25/00

(54) **SPIROCYCLIC DERIVATIVE-CONTAINING SALT OR CRYSTAL FORM, AND PREPARATION METHOD THEREFOR AND USE THEREOF**

(30) Priority: 27.09.2022 CN 202211181361
(71) Applicant: Shujing Biopharma Co., Ltd, Shanghai 201208 (CN); Jiangsu NHWA Pharmaceutical Co., Ltd, Xuzhou, Jiangsu 221000 (CN)
(72) Inventor: LIANG, Xiaoxiao, Shanghai 201208 (CN); WU, Yongqi, Shanghai 201208 (CN); WANG, Jin, Shanghai 201208 (CN); MA, Shihan, Shanghai 201208 (CN); ZHOU, Bingcheng, Shanghai 201208 (CN); QIN, Jun, Shanghai 201208 (CN); WAN, Zehong, Shanghai 201208 (CN)
(74) Representative: Lavoix
(86) International application number: PCT/CN2023/121363
(87) International publication number: WO 2024/067542

(57) **Abstract**

The present invention relates to a spirocyclic derivative-containing salt or crystal form, and a preparation method therefor and a use thereof. Particularly, the present invention relates to a salt or crystal form of a compound (R)-1-(4'H,6'H-spiro[cyclopropane-1,7'-thieno[3,2-c]pyran]-4'-yl)-N-methylmethyla-mine, a preparation method therefor and a use thereof, and a pharmaceutical composition of the salt or crystal form containing a therapeutically effective amount of the compound and a use thereof in preparation of a drug for preventing and/or treating neuropsychiatric disorders.

## Description

The present application claims priority of Chinese patent application No.2022111813611 filed on September 27, 2022. The present application refers to the full text of the above-mentioned China patent application.

### TECHNICAL FIELD

The present application belongs to the field of pharmaceutical synthesis technology, in particular to a salt, a crystal form of a spirocycle-containing derivative, a preparation method and use thereof.

### BACKGROUND

Diseases related to central nervous system affect a large population in varying degrees. In general, the main features of such diseases include marked impairment of cognition or memory, and a marked deterioration in relative to the original level of function. Schizophrenia is a psychopathic disorder of unknown origin that usually begins by early adulthood, characterized by psychotic symptoms, stage progression and development, and/or regression in social behavior and professional ability. Symptoms of schizophrenia are generally manifested in three major categories: positive symptoms, negative symptoms, and cognitive symptoms. Positive symptoms are symptoms that manifest as "excessive" normal experiences, such as hallucinations and delusions. Negative symptoms are symptoms of a lack of normal experiences, such as anhedonia and a lack of social interaction. Cognitive symptoms, such as lack of sustained attention and poor decision-making, are associated with cognitive impairment in schizophrenia. Current anti-psychotic drugs can successfully treat positive symptoms, but are far from ideal for negative and cognitive symptoms.

Biogenic amines play important roles as neurotransmitters in the central and peripheral nervous systems. The synthesis and storage of biogenic amines, as well as their degradation and reabsorption after release, are tightly controlled. Imbalances in biogenic amine levels are known to be a major cause of brain function changes in many pathological conditions. Serotonin, norepinephrine, epinephrine, dopamine, and histamine have been extensively studied as classic biogenic amines. Particularly, the 5-hydroxytryptamine system plays an important role in regulating the functions of prefrontal cortex (PFC), including emotional control, cognitive behavior, and working memory. The pyramidal neurons and GABA interneurons of PFC comprise several 5-hydroxytryptamine receptor subtypes 5-HT1A and 5-HT2A with particularly high density. It has recently been demonstrated that PFC and NMDA receptor channels are targets of 5-HT1AR, and these two receptors regulate excitatory neurons in the cerebral cortex, thereby affecting cognitive function. In fact, various preclinical data indicate that 5-HT1AR may be a new target for the development of anti-psychotic drugs. The high affinity of atypical anti-psychotic drugs (such as olanzapine, aripiprazole.) to 5-HT1AR and their low EPS side effects indicate that the 5-hydroxytryptamine system plays an important role in regulating the functions of PFC, including emotional control, cognitive behavior and working memory. The pyramidal neurons and GABA interneurons of PFC comprise several 5-hydroxytryptamine receptor subtypes 5-HT1A and 5-HT2A with particularly high density. Recent studies have shown that 5-HT1A agonists are associated with atypical anti-psychotic drugs treatment and can improve negative symptoms and cognitive disorder.

In recent years, with the gradual deepening of the research on classical biogenic amines, people have found a second type of endogenous amine compounds, namely trace amines TA, including p-tyramine, β-phenylethylamine, tryptamine and octopamine. The content level of these compounds in the mammalian nervous system is generally lower than that of classical biogenic amines, but they share similar characteristics with classical biogenic amines in terms of structure, metabolism, and subcellular localization. Trace amine-associated receptor TAAR, a new member of G protein-coupled receptors (GPCR), shares similar structure and consistent pharmacological data with the pharmacophore penetrating into GPCR. The phylogenetic relationship of this receptor gene indicates that these receptors form three distinct subfamilies, of which TAAR1 is the first subfamily of the four genes (TAAR1-4) highly conserved between humans and rodents. TA activates TAAR1 through Gαs and plays a role. Existing studies have shown that dysregulation of trace amine-associated receptors, especially TAAR1, is closely related to many psychiatric diseases such as schizophrenia and depression as well as other conditions such as attention deficit hyperactivity disorder, migraine, Parkinson's disease, substance abuse and eating disorder. Therefore, TAAR ligands have high potential for the treatment of these diseases.

Although there are many anti-schizophrenia drugs, there are still a variety of adverse reactions of schizophrenic drugs in clinical application. In addition, although the current anti-schizophrenia negative symptom drugs have been applied clinically and improved the negative symptoms in some patients, the overall effect is limited. There are still many patients who cannot recover and repair normal social functions due to negative symptoms, resulting in difficulties in restoring normal social work. In addition, the treatment of cognitive disorder is also a key point in the treatment of schizophrenia at present, which affects the verbal memory, semantic processing ability and attention function of most patients with schizophrenia. However, the improvement of cognitive function by anti-psychotic drugs currently being researched or marketed is very limited. In addition, the treatment of refractory schizophrenia is still in a dilemma. Such patients have been treated with three anti-psychotic drugs with different active ingredients, with a sufficient quantity of drugs and sufficient treatment duration but poor treatment response or inability to tolerate adverse reactions of drugs, or even with sufficient maintenance or preventive treatment, the condition continues to recur or worsen. Therefore, therapeutic drugs for refractory schizophrenia have always been a challenge in clinical drug research and an urgent direction to be overcome.

Currently, Sunovion's SEP-363856, which is in phase III clinical research, as a 5-hydroxytryptamine and/or trace amine-associated receptor agonist, has significant effects on 5-HT1A and TAAR1 receptors and has shown good activity in present clinical study. Therefore, there is an urgent need to develop an anti-schizophrenia drug that has good, sustained and effective treatment effects for negative symptoms, can improve cognitive function of patients, can effectively treat refractory schizophrenia, and in addition, has low adverse drug reactions and acts on multiple targets, in order to meet the huge market demand.

### SUMMARY

The international patent application PCT/CN2022/083485 describes a series of structures of spirocycle-containing derivatives. In subsequent research and development, in order to improve the solubility, stability, powder flowability, pharmacokinetics, and/or bioavailability of the compounds, reduce storage costs, and extend product cycles, the present application has carried out a comprehensive study of the acid salts and crystal forms of the aforementioned compounds.

All contents related to the international patent application PCT/CN2022/083485 are added to the present application by reference.

The technical problem to be solved by the present application is to provide a salt, a crystal form of a spirocycle-containing derivative, and a preparation method and use thereof.

The purpose of the present application is to provide an acid salt of a compound (R)-1-(4'H,6'H-spiro[cyclopropane-1,7'-thieno[3,2-c]pyran]-4'-yl)-N-methylmethyl-amine.

In a preferred embodiment of the present application, the acid salt is a hydrochloride salt.

In a preferred embodiment of the present application, the number of acid molecules in the acid salt is 0.2-3, preferably 0.2, 0.5, 1, 1.5, 2, 2.5 or 3, more preferably 0.5, 1, 2 or 3, and further preferably 1.

In a further preferred embodiment of the present application, the molar ratio of the compound (R)-1-(4'H,6'H-spiro[cyclopropane-1,7'-thieno[3,2-c]pyran]-4'-yl)-N-methylmethyl-amine to hydrochloride salt is 1:0.8-1.2.

In a further preferred embodiment of the present application, the acid salt is a non-solvate or a solvate, wherein the solvent is one or more selected from the group consisting of: water, methanol, ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, and toluene.

In a further preferred embodiment of the present application, the number of solvent molecules mentioned above is 0-3, preferably 0, 0.2, 0.5, 1, 1.5, 2, 2.5 or 3, and more preferably 0, 0.5, 1, 2 or 3.

In a further preferred embodiment of the present application, the acid salt is preferably an anhydrate or a monohydrate.

In a further preferred embodiment of the present application, the acid salt is in crystalline or amorphous form.

In a further preferred embodiment of the present application, the acid salt of the compound (R)-1-(4'H,6'H-spiro[cyclopropane-1,7'-thieno[3,2-c]pyran]-4'-yl)-N-methylmethylamine is hydrochloride crystal form I, hydrochloride crystal form II, or hydrochloride crystal form III, wherein:
the number of the acid molecules in the hydrochloride crystal form I is 1, and the X-ray powder diffraction spectrum comprises diffraction peaks located at 2θ of 12.03 ± 0.2°, 16.08 ± 0.2°, and 26.84 ± 0.2°;
preferably, it comprises diffraction peaks located at 2θ of 12.03 ± 0.2°, 16.08 ± 0.2°, 18.52 ± 0.2°, 25.31 ± 0.2°, and 26.84 ± 0.2°;
preferably, it comprises diffraction peaks located at 2θ of 12.03 ± 0.2°, 16.08 ± 0.2°, 18.52 ± 0.2°, 20.94 ± 0.2°, 22.06 ± 0.2°, 23.10 ± 0.2°, 24.72 ± 0.2°, 25.31 ± 0.2°, 26.84 ± 0.2°, and 29.60 ± 0.2°;
more preferably, it comprises diffraction peaks located at 2θ of 6.22 ± 0.2°, 12.03 ± 0.2°, 12.36 ± 0.2°, 16.08 ± 0.2°, 18.25 ± 0.2°, 18.52 ± 0.2°, 19.48 ± 0.2°, 20.24 ± 0.2°, 20.94 ± 0.2°, 21.18 ± 0.2°, 22.06 ± 0.2°, 23.10 ± 0.2°, 23.54 ± 0.2°, 24.72 ± 0.2°, 25.31 ± 0.2°, 26.84 ± 0.2°, 27.07 ± 0.2°, 27.77 ± 0.2°, 28.49 ± 0.2°, 29.60 ± 0.2°, 31.05 ± 0.2°, 31.78 ± 0.2°, 32.45 ± 0.2°, 32.79 ± 0.2°, 37.15 ± 0.2°, 39.10 ± 0.2°, 42.73 ± 0.2°, 43.97 ± 0.2°, 44.36 ± 0.2°, and 45.23 ± 0.2°;
further preferably, the X-ray diffraction peaks represented by 2θ angle and interplanar spacing d value using Cu-Kα radiation are as shown in Table 1.

**Table 1: X-ray powder diffraction data for crystal form I of the hydrochloride crystal form I of the compound**

| Serial number | 2θ(±0.2°) | d value | Relative strength | Serial number | 2θ(±0.2°) | d value | Relative strength |
|---|---|---|---|---|---|---|---|
| 1 | 6.22 | 14.203 | 21.7% | 16 | 26.84 | 3.319 | 62.2% |
| 2 | 12.03 | 7.348 | 68.9% | 17 | 27.07 | 3.291 | 26.6% |
| 3 | 12.36 | 7.157 | 28.2% | 18 | 27.77 | 3.210 | 21.3% |
| 4 | 16.08 | 5.509 | 100.0% | 19 | 28.49 | 3.130 | 10.9% |
| 5 | 18.25 | 4.858 | 26.4% | 20 | 29.60 | 3.015 | 34.8% |
| 6 | 18.52 | 4.787 | 43.2% | 21 | 31.05 | 2.878 | 11.5% |
| 7 | 19.48 | 4.553 | 26.1% | 22 | 31.78 | 2.814 | 9.2% |
| 8 | 20.24 | 4.383 | 19.5% | 23 | 32.45 | 2.757 | 10.5% |
| 9 | 20.94 | 4.239 | 29.9% | 24 | 32.79 | 2.729 | 10.7% |
| 10 | 21.18 | 4.192 | 17.7% | 25 | 37.15 | 2.418 | 7.6% |
| 11 | 22.06 | 4.026 | 37.2% | 26 | 39.10 | 2.302 | 8.8% |
| 12 | 23.10 | 3.847 | 28.5% | 27 | 42.73 | 2.114 | 9.7% |
| 13 | 23.54 | 3.777 | 27.8% | 28 | 43.97 | 2.058 | 7.3% |
| 14 | 24.72 | 3.598 | 44.3% | 29 | 44.36 | 2.040 | 8.2% |
| 15 | 25.31 | 3.516 | 48.1% | 30 | 45.23 | 2.003 | 11.7% |

Still further preferably, the X-ray powder diffraction spectrum of hydrochloride crystal form I of the compound (R)-1-(4'H,6'H-spiro[cyclopropane-1,7'-thieno[3,2-c]pyran]-4'-yl)-N-methylmethyl-amine is basically as shown in Fig. 1; its DSC diagram is basically as shown in Fig.2; and its TGA diagram is basically shown in Fig.3.

The number of the acid molecules in the hydrochloride crystal form II is 1, and its X-ray powder diffraction spectrum comprises diffraction peaks located at 2θ of 13.36 ± 0.2°, 17.25 ± 0.2°, and 27.74 ± 0.2°;
preferably, it comprises diffraction peaks located at 2θ of 10.30 ± 0.2°, 13.36 ± 0.2°, 17.25 ± 0.2°, 22.77 ± 0.2°, and 27.74 ± 0.2°;
preferably, it comprises diffraction peaks located at 2θ of 10.30 ± 0.2°, 13.36 ± 0.2°, 15.17 ± 0.2°, 17.25 ± 0.2°, 22.77 ± 0.2°, 24.95 ± 0.2°, 25.28 ± 0.2°, 25.87 ± 0.2°, 27.74 ± 0.2°, and 30.99 ± 0.2°;
more preferably, it comprises diffraction peaks located at 2θ of 8.55 ± 0.2°, 9.96 ± 0.2°, 10.30 ± 0.2°, 11.23 ± 0.2°, 12.49 ± 0.2°, 13.36 ± 0.2°, 15.17 ± 0.2°, 15.99 ± 0.2°, 17.25 ± 0.2°, 18.25 ± 0.2°, 18.83 ± 0.2°, 19.20 ± 0.2°, 20.04 ± 0.2°, 21.11 ± 0.2°, 21.72 ± 0.2°, 22.77 ± 0.2°, 24.13 ± 0.2°, 24.95 ± 0.2°, 25.28 ± 0.2°, 25.87 ± 0.2°, 26.39 ± 0.2°, 27.11 ± 0.2°, 27.74 ± 0.2°, 28.57 ± 0.2°, 29.23 ± 0.2°, 30.99 ± 0.2°, 32.26 ± 0.2°, 34.85 ± 0.2°, 38.72 ± 0.2°, and 40.42 ± 0.2°;
further preferably, the X-ray diffraction peaks represented by 2θ angle and interplanar spacing d value using Cu-Kα radiation are as shown in Table 2.

**Table 2: X-ray powder diffraction data for hydrochloride crystal form II of the compound**

| Serial number | 2θ(±0.2°) | d value | Relative strength | Serial number | 2θ(±0.2°) | d value | Relative strength |
|---|---|---|---|---|---|---|---|
| 1 | 8.55 | 10.338 | 4.8% | 16 | 22.77 | 3.903 | 11.8% |
| 2 | 9.96 | 8.873 | 6.5% | 17 | 24.13 | 3.685 | 5.5% |
| 3 | 10.30 | 8.581 | 11.1% | 18 | 24.95 | 3.566 | 10.1% |
| 4 | 11.23 | 7.874 | 3.8% | 19 | 25.28 | 3.520 | 7.6% |
| 5 | 12.49 | 7.080 | 5.7% | 20 | 25.87 | 3.441 | 8.5% |
| 6 | 13.36 | 6.621 | 52.1% | 21 | 26.39 | 3.375 | 6.0% |
| 7 | 15.17 | 5.835 | 8.7% | 22 | 27.11 | 3.287 | 7.0% |
| 8 | 15.99 | 5.539 | 3.8% | 23 | 27.74 | 3.213 | 17.7% |
| 9 | 17.25 | 5.138 | 100.0% | 24 | 28.57 | 3.122 | 5.8% |
| 10 | 18.25 | 4.856 | 5.9% | 25 | 29.23 | 3.053 | 4.7% |
| 11 | 18.83 | 4.709 | 6.2% | 26 | 30.99 | 2.884 | 8.4% |
| 12 | 19.20 | 4.618 | 4.7% | 27 | 32.26 | 2.772 | 4.1% |
| 13 | 20.04 | 4.427 | 4.7% | 28 | 34.85 | 2.573 | 4.2% |
| 14 | 21.11 | 4.205 | 6.4% | 29 | 38.72 | 2.324 | 2.9% |
| 15 | 21.72 | 4.088 | 5.4% | 30 | 40.42 | 2.230 | 3.4% |

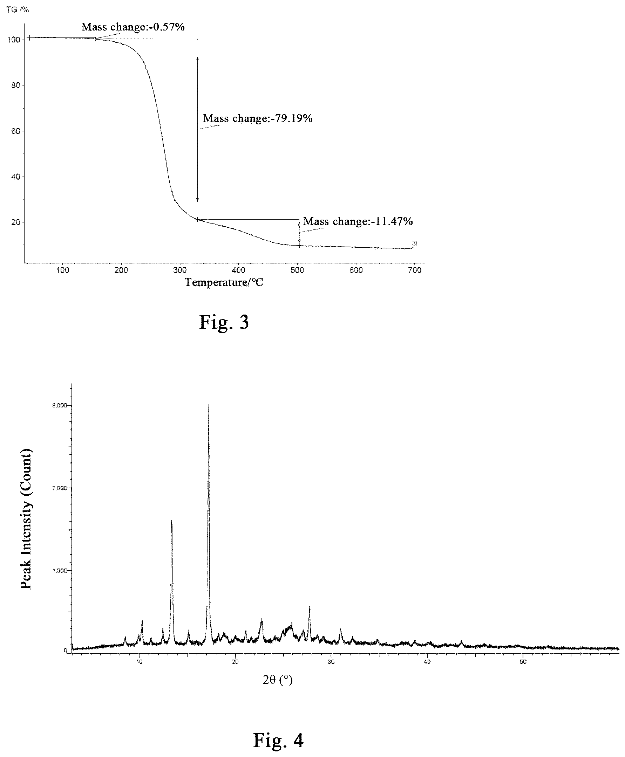

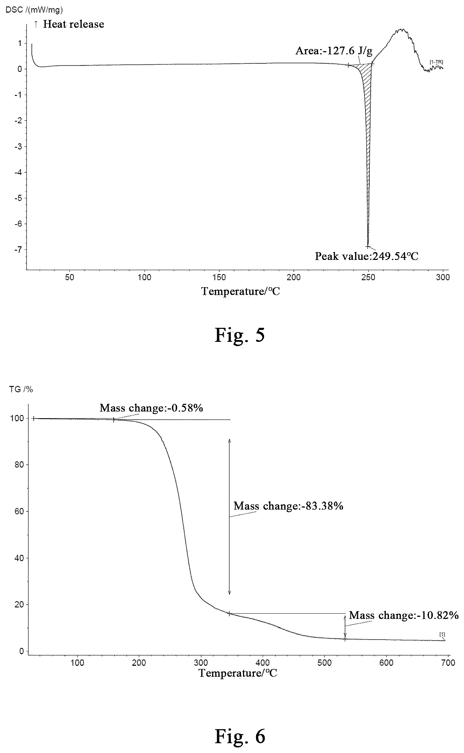

Still further preferably, the X-ray powder diffraction spectrum of hydrochloride crystal form II of the compound (R)-1-(4'H,6'H-spiro[cyclopropane-1,7'-thieno[3,2-c]pyran]-4'-yl)-N-methylmethyl-amine is as shown in Fig.4; its DSC diagram is basically as shown in Fig.5; and its TGA diagram is basically as shown in Fig.6.

The number of the acid molecules in the hydrochloride crystal form III is 1, and its X-ray powder diffraction spectrum comprises diffraction peaks located at 2θ of 8.86 ± 0.2°, 17.71 ± 0.2°, and 26.66 ± 0.2°;
preferably, it comprises diffraction peaks located at 2θ of 8.86 ± 0.2°, 13.28 ± 0.2°, 17.71 ± 0.2°, 19.07 ± 0.2°, and 26.66 ± 0.2°;
preferably, it comprises diffraction peaks located at 2θ of 8.86 ± 0.2°, 13.28 ± 0.2°, 15.75 ± 0.2°, 16.22 ± 0.2°, 17.71 ± 0.2°, 19.07 ± 0.2°, 22.75 ± 0.2°, 23.70 ± 0.2°, 26.66 ± 0.2°, and 31.19 ± 0.2°;
more preferably, it comprises diffraction peaks located at 2θ of 8.86 ± 0.2°, 10.50 ± 0.2°, 12.99 ± 0.2°, 13.28 ± 0.2°, 15.75 ± 0.2°, 16.22 ± 0.2°, 17.71 ± 0.2°, 19.07 ± 0.2°, 20.52 ± 0.2°, 21.01 ± 0.2°, 22.75 ± 0.2°, 23.19 ± 0.2°, 23.70 ± 0.2°, 24.16 ± 0.2°, 24.43 ± 0.2°, 24.91 ± 0.2°, 25.34 ± 0.2°, 25.70 ± 0.2°, 26.09 ± 0.2°, 26.66 ± 0.2°, 27.19 ± 0.2°, 29.06 ± 0.2°, 31.01 ± 0.2°, 31.19 ± 0.2°, 31.41 ± 0.2°, 31.67 ± 0.2°, 32.07 ± 0.2°, 32.98 ± 0.2°, 33.40 ± 0.2°, 36.77 ± 0.2°, 44.77 ± 0.2°, and 49.93 ± 0.2°;
further preferably, the X-ray diffraction peaks represented by 2θ angle and interplanar spacing d value using Cu-Kα radiation are as shown in Table 3.

**Table 3: X-ray powder diffraction data for hydrochloride crystal form III of the compound**

| Serial number | 2θ(±0.2°) | d value | Relative strength | Serial number | 2θ(±0.2°) | d value | Relative strength |
|---|---|---|---|---|---|---|---|
| 1 | 8.86 | 9.970 | 33.4% | 17 | 25.34 | 3.512 | 6.8% |
| 2 | 10.50 | 8.416 | 2.4% | 18 | 25.70 | 3.464 | 5.4% |
| 3 | 12.99 | 6.812 | 1.9% | 19 | 26.09 | 3.412 | 5.1% |
| 4 | 13.28 | 6.664 | 13.6% | 20 | 26.66 | 3.341 | 30.8% |
| 5 | 15.75 | 5.621 | 9.9% | 21 | 27.19 | 3.277 | 5.3% |
| 6 | 16.22 | 5.461 | 8.1% | 22 | 29.06 | 3.071 | 7.6% |
| 7 | 17.71 | 5.005 | 100.0% | 23 | 31.01 | 2.882 | 3.3% |
| 8 | 19.07 | 4.650 | 15.1% | 24 | 31.19 | 2.865 | 9.8% |
| 9 | 20.52 | 4.325 | 3.0% | 25 | 31.41 | 2.846 | 4.2% |
| 10 | 21.01 | 4.225 | 3.7% | 26 | 31.67 | 2.823 | 2.7% |
| 11 | 22.75 | 3.906 | 10.3% | 27 | 32.07 | 2.789 | 2.9% |
| 12 | 23.19 | 3.833 | 5.8% | 28 | 32.98 | 2.714 | 2.9% |
| 13 | 23.70 | 3.752 | 8.7% | 29 | 33.40 | 2.681 | 2.8% |
| 14 | 24.16 | 3.680 | 2.7% | 30 | 36.77 | 2.442 | 2.3% |
| 15 | 24.43 | 3.641 | 3.8% | 31 | 44.77 | 2.023 | 2.5% |
| 16 | 24.91 | 3.572 | 2.6% | 32 | 49.93 | 1.825 | 2.3% |

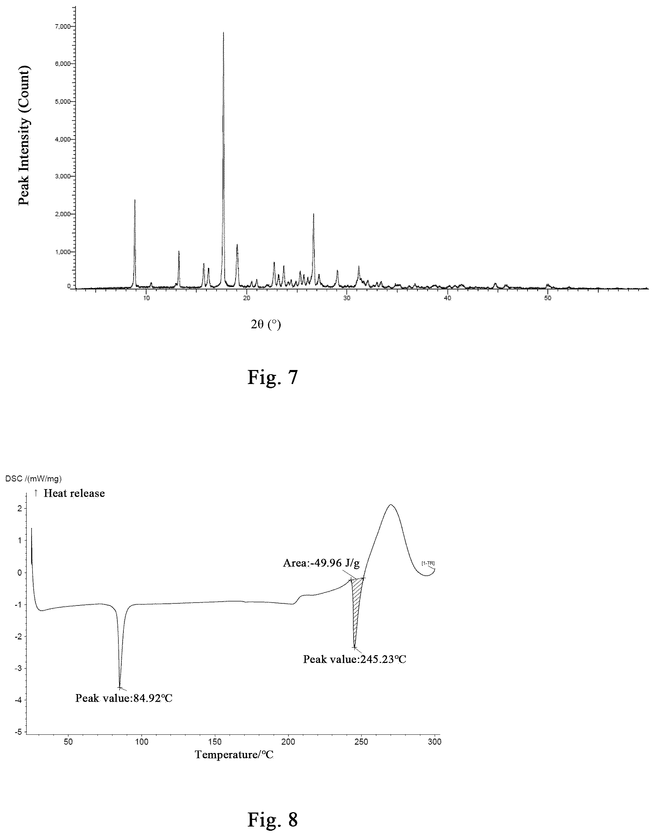

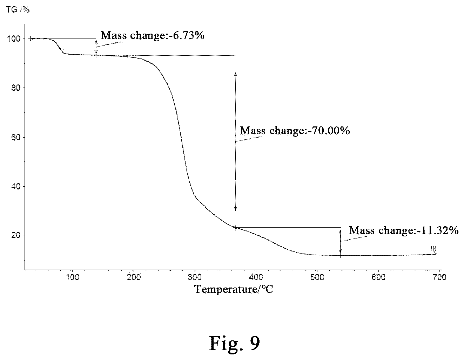

Still further preferably, the X-ray powder diffraction spectrum of hydrochloride crystal form III of the compound (R)-1-(4'H,6'H-spiro[cyclopropane-1,7'-thieno[3,2-c]pyran]-4'-yl)-N-methylmethyl-amine is shown in Fig.7; its DSC diagram is basically as shown in Fig.8; and its TGA diagram is basically as shown in Fig.9.

On the other hand, the present application also relates to a method for preparing an acid salt of the compound (R)-1-(4'H,6'H-spiro[cyclopropane-1,7'-thieno[3,2-c]pyran]-4'-yl)-N-methylmethyl-amine, comprising the following steps:
(1) weighing an appropriate amount of free alkali of the compound (R)-1-(4'H,6'H-spiro[cyclopropane-1,7'-thieno[3,2-c]pyran]-4'-yl)-N-methylmethyl-amine, and dissolving it with solvent 1;
(2) weighing an appropriate amount of hydrochloric acid, and dissolving it with solvent 2; preferably the amount of the hydrochloric acid is 0.5-2.0 equivalents;
(3) mixing the above two solutions, stirring and reacting at a certain temperature for a certain period of time, filtering with suction, and drying to obtain a target product; or

(1) weighing an appropriate amount of free alkali of the compound (R)-1-(4'H,6'H-spiro[cyclopropane-1,7'-thieno[3,2-c]pyran]-4'-yl)-N-methylmethyl-amine, and dissolving it with solvent 1;
(2) weighing an appropriate amount of hydrochloric acid and dissolving it in solvent 2; preferably the amount of the hydrochloric acid is 0.5-2.0 equivalents;
(3) mixing the above two solutions, and stirring and reacting at a certain temperature for a certain period of time, adding solvent 3 to continue stirring and reacting for a certain period of time, filtering with suction, and drying to obtain a target product;

wherein the reaction temperature is determined by the solvent in the system, and is preferably room temperature;
the solvents 1, 2, and 3 are independently selected from the group consisting of: water, methanol, ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, and toluene; wherein solvent 1 and solvent 2 need to be miscible when used.

On the other hand, the present application also relates to a method for preparing an acid salt of the compound (R)-1-(4'H,6'H-spiro[cyclopropane-1,7'-thieno[3,2-c]pyran]-4'-yl)-N-methylmethyl-amine, including the following steps:
(1) weighing an appropriate amount of the hydrochloride of the compound, and dissolving or suspending it with solvent 4;
(2) refluxing the clarified solution or suspension from obtained in step (1) to react at a certain temperature for a certain period of time, cooling to room temperature, filtering with suction, and drying to obtain a target product;

wherein the reflux temperature is generally slightly higher than the boiling point temperature of solvent 4, preferably 70-90°C, more preferably 75-85°C, and further preferably 80°C;
the solvent 4 is selected from the group consisting of: water, anhydrous methanol, anhydrous ethanol, 95% ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, and toluene.

The present application further relates to a pharmaceutical composition comprising a therapeutically effective amount of an acid salt of any of the compounds shown or a combination thereof, and one or more pharmaceutically acceptable carriers, diluents, or excipients.

The present application further relates to a use of any acid salt of the compound shown, or its pharmaceutical composition in the preparation of a medicament.

In a further preferred embodiment of the present application, the medicament can be a medicament for the preventing and/or treating a neuropsychiatric disease in mammals.

In a further preferred embodiment of the present application, the neuropsychiatric disease is preferably a central nervous system disease related to 5-hydroxytryptamine receptors and/or trace amine-associated receptors and/or dopamine receptors.

In a further preferred embodiment of the present application, the 5-hydroxytryptamine receptor is preferably a 5-HT_{1A} receptor.

In a further preferred embodiment of the present application, the trace amine-associated receptor is preferably a TAAR1 receptor.

In a further preferred embodiment of the present application, the neuropsychiatric disease is one or more diseases selected from the group consisting of: schizophrenia, schizophrenia spectrum disease, acute schizophrenia, chronic schizophrenia, NOS schizophrenia, schizoid personality disorder, schizotypal personality disorder, delusional disorder, psychosis, psychotic disorder, brief psychotic disorder, shared psychotic disorder, psychotic disorder due to a physical disease, drug-induced psychosis, psychoaffective disorder, aggression, delirium, Parkinson's psychosis, excitative psychosis, Tourette's syndrome, organic or NOS psychosis, seizure, agitation, post-traumatic stress disorder, behavior disorder, neurodegenerative disease, Alzheimer's disease, Parkinson's disease, dyskinesias, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse or dependence, addiction, eating disorder, sexual dysfunction, hypertension, emesis, Lesche-Nyhane disease, Wilson's disease, autism, Huntington's chorea, and premenstrual dysphoria.

### DETAILED DESCRIPTION

Different terms such as "X is selected from A, B, or C", "X is selected from A, B, and C", "X is A, B, or C", "X is A, B, and C", etc., all express the same meaning, that is X can be any one or more of A, B, and C.

"Optional" or "optionally" means that the subsequently described event or situation may or may not occur, and the expression includes situations where the event or environment occurs or does not occur. For example, "cycloalkyl optionally substituted with alkyl" means that alkyl may but does not have to exist, including the situation where cycloalkyl is substituted with alkyl and the situation where cycloalkyl is not substituted with alkyl.

"Pharmaceutical composition" refers to a mixture of one or more compounds of the present application or a physiologically/pharmaceutically acceptable salt or prodrug thereof and other chemical components, as well as other components such as physiologically/pharmaceutically acceptable carriers, diluents, or excipients. The purpose of a pharmaceutical composition is to promote the administration of drugs to organisms, facilitate the absorption of active ingredients, and thus exert biological activity.

"Pharmaceutically acceptable salts" refer to the salts of the compound of the present application, which are safe, effective, and have the expected biological activity when used in mammals.

As used herein, "polymorphs" refers to crystal forms with the same chemical composition but different spatial arrangements of molecules, atoms, and/or ions that constitute the crystal. Although polymorphs have the same chemical composition, their stacking and geometric arrangement are different, and they may exhibit different physical properties such as melting point, shape, color, density, hardness, deformability, stability, solubility, dissolution rate, and the like. According to their temperature-stability relationship, the relative stability between the two solid phases is swapped. The phenomenon of this compound existing in different lattice structures is called drug polymorphism.

Crystal structures equivalent to those disclosed or claimed in the present application may exhibit similar but not identical analytical characteristics within a reasonable error range according to experimental conditions, purity, equipment, and other common variables known to those skilled in the art. Correspondingly, it is obvious to those skilled in the art that various modifications and variations can be made within the scope and spirit of the present application. Based on the specification and practice of the application disclosed herein, other embodiments of the present application will be apparent to those skilled in the art. The applicant expects the specification and examples are to be considered exemplary rather than limiting their scope.

"X-ray powder diffraction spectrum or XRPD" refers to the diffraction spectrum obtained according to Bragg equation 2d sin θ = nλ (where λ is the wavelength of the X-rays, λ = 1.54056 Å, and the diffraction order n is any positive integer, typically take the first-order diffraction peak, n = 1); when X-ray is incident on an atomic plane with a lattice plane spacing of d in a crystal or part of a crystal sample at a grazing angle θ (the complementary angle of the incident angle, also known as the Bragg angle), the Bragg equation is satisfied, and this X-ray powder diffraction spectrum can be measured. The "2θ or 2θ angle" refers to the diffraction angle, wherein θ is the Bragg angle, measured in degrees (°).

As is well known to those skilled in the art, XRPD may have certain displacement and intensity deviation due to factors such as the tile thickness of the sample, detection methods, conditions, and instruments. The same Samples of the same crystal form usually have the same main XRPD characteristic peaks, but there may be some operational errors. When samples of the same crystal form obtained by corresponding methods by a person skilled in the art are detected using the same instrument and detection method, the characteristic peak error is usually within ± 0.2°. However, the error of a few characteristic peaks may occasionally exceed this range when different technicians use different instruments, and if the error is within ±0.5° or ±0.3°, they should be considered as XRPD characteristic peaks of the same crystal form. Therefore, as a specific example of the crystal form of the present application, its XRPD is shown in spectrum X. However, a person skilled in the art understand that when the deviation of the key characteristic peak displacement 2θ is within ± 0.5°, ± 0.3°, or ± 0.2°, especially around ± 0.2°, it can be recognized as the same crystal form and can be interpreted as being within the protection scope of the present application.

In addition, the absolute intensity and relative intensity of the peaks shown in the aforementioned tables and figures may vary due to various factors, such as the effect of the selected orientation of the crystalline solid on the X-ray beam, the influence of coarse particles, the purity of the analyzed substance, or the crystallinity of the sample. In addition, the peak position can also shift according to the change in the sample height. Furthermore, if different wavelengths are used for measurement, different displacement values can be obtained based on the Bragg equation (n λ=2dsin θ), and such different XRPD spectrums obtained by using different wavelengths are also included in the scope of the present application.

"Interplanar spacing or interplanar spacing (d value)" refers to the selection of 3 non-parallel unit vectors a, b, c that connect two adjacent lattice points in a spatial lattice, which divide the lattice into juxtaposed parallelepiped units, known as interplanar spacing. The space lattice is divided according to the determined parallelepiped unit connection line to obtain a set of linear grids, which is called space lattice or crystal lattice. Lattice and crystal lattice reflect the periodicity of crystal structure with geometric points and lines respectively. Different crystal planes have different interplanar spacing (i.e. the distance between two adjacent parallel crystal planes); the unit is Å or angstrom.

"Relative intensity (I%)" refers to the ratio of the intensity of the other peak to the intensity of the first strong peak among all diffraction peaks in the X-ray powder diffraction pattern (XRPD) when the intensity of the first strong peak is 100%.

"Differential Scanning Calorimetry analysis or DSC" determines the transition temperature at which a crystal absorbs or releases heat due to changes in its crystal structure or the melting of the crystal. For the same crystal form of the same compound, in continuous analysis, the error of the thermal transition temperature and melting point can be within about 5°C, usually within about 3°C. When a compound is described as having a given DSC peak or melting point, it refers to the DSC peak or melting point being ± 5°C, and this temperature variation is basically taken into account. DSC provides an auxiliary method for distinguishing different crystal forms. Different crystal morphologies can be identified based on their distinct transition temperature characteristics. It should be pointed out that for mixtures, their DSC peaks or melting points may vary over a larger range. In addition, since decomposition is accompanied by the melting process of a substance, the melting temperature is related to the heating rate.

"Thermogravimetric analysis (TGA)" is a common method for determining the thermal stability of compounds. In the present application, TGA can also be used to determine the hydration state of compounds, and the heating rate will have a certain impact on the spectrum during the testing process. The error of TGA can be within about ± 0.5% by mass.

"Amorphous" or "amorphous form" refers to the substance formed when the particles (molecules, atoms, ions) of a substance are arranged aperiodically in three-dimensional space, characterized by a diffused X-ray powder diffraction spectrum without sharp peaks. Amorphousness is a special physical form of solid matter, and its locally ordered structural characteristics suggest that it is aperiodically linked with crystalline substances.

"Equivalent" or its abbreviation "eq" refers to the equivalent amount of other raw materials required based on the basic raw materials used in each step (1 equivalent) according to the equivalent relationship of chemical reactions.

"Basically as shown in the figure" refers to at least 50%, or at least 60%, or at least 70%, or at least 80%, or at least 90%, or at least 95%, or at least 99% of the peaks in the X-ray powder diffraction spectrum, DSC diagram, Raman spectrum, or infrared spectrum being shown in the figure.

In the context of the present application, when or whether the word "approximately" or "about" is used, it means within 10% of a given value or range, appropriately within 5%, and especially within 1%. Alternatively, for those skilled in the art, the term "approximately" or "about" means within an acceptable standard error range of the mean value. Whenever a number with a value of N is disclosed, any number with N+/-1%, N+/-2%, N+/-3%, N+/-5%, N+/-7%, N+/-8%, or N+/-10% is explicitly disclosed, wherein "+/-" refers to addition or subtraction.

"Room temperature" refers to a temperature ranging from 10°C to 40°C. In some embodiments, "room temperature" refers to a temperature ranging from 15°C to 30°C; in other embodiments, "room temperature" refers to a temperature ranging from 18°C to 25°C.

### Beneficial effects

The hydrochloride crystal form of the compound of the present application not only performs well in product performance parameters such as melting point, solubility, solution stability and solid stability, but also shows obvious advantages in bioavailability.

### BRIEF DESCRIPTION OF THE DRAWINGS

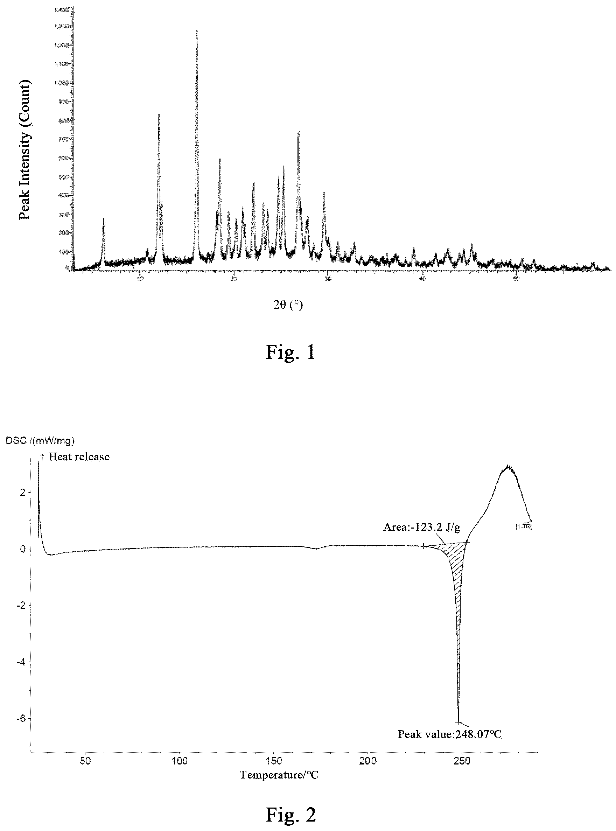

Fig.1 shows the XRPD spectrum of the hydrochloride crystal form I of (R)-1-(4'H,6'H-spiro[cyclopropane-1,7'-thieno[3,2-c]pyran]-4'-yl)-N-methylmethyl-amine.
Fig.2 shows the DSC diagram of the hydrochloride crystal form I of (R)-1-(4'H,6'H-spiro[cyclopropane-1,7'-thieno[3,2-c]pyran]-4'-yl)-N-methylmethyl-amine.
Fig.3 shows the TGA diagram of the hydrochloride crystal form I of (R)-1-(4'H,6'H-spiro[cyclopropane-1,7'-thieno[3,2-c]pyran]-4'-yl)-N-methylmethyl-amine.
Fig.4 shows the XRPD spectrum of the hydrochloride crystal form II of (R)-1-(4'H,6'H-spiro[cyclopropane-1,7'-thieno[3,2-c]pyran]-4'-yl)-N-methylmethyl-amine.
Fig.5 shows the DSC diagram of the hydrochloride crystal form II of (R)-1-(4'H,6'H-spiro[cyclopropane-1,7'-thieno[3,2-c]pyran]-4'-yl)-N-methylmethyl-amine.
Fig.6 shows the TGA diagram of the hydrochloride crystal form II of (R)-1-(4'H,6'H-spiro[cyclopropane-1,7'-thieno[3,2-c]pyran]-4'-yl)-N-methylmethyl-amine.
Fig.7 shows the XRPD spectrum of the hydrochloride crystal form III of (R)-1-(4'H,6'H-spiro[cyclopropane-1,7'-thieno[3,2-c]pyran]-4'-yl)-N-methylmethyl-amine.
Fig.8 shows the DSC diagram of the hydrochloride crystal form III of (R)-1-(4'H,6'H-spiro[cyclopropane-1,7'-thieno[3,2-c]pyran]-4'-yl)-N-methylmethyl-amine.
Fig.9 shows the TGA diagram of the hydrochloride crystal form III of (R)-1-(4'H,6'H-spiro[cyclopropane-1,7'-thieno[3,2-c]pyran]-4'-yl)-N-methylmethyl-amine.

### DETAILED DESCRIPTION

The present application is further described below in combination with specific examples. It should be understood that these examples are only used to illustrate the present application and are not intended to limit the scope of the present application. In addition, it should be understood that after reading the content of the present application, those skilled in the art may make various changes or modifications to the present application, and these equivalent forms also fall within the scope defined by the appended claims of the present application.

The following SEP-363856 of the present application is as follows, and it was prepared with reference to the method of Example 129 of patent PCT/US2010/058884,

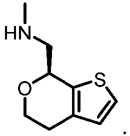

The following comparative example 1 of the present application is as follows, and it was prepared with reference to the method of Example 89 of patent PCT/US2010/058884,

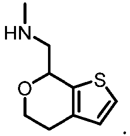

### I. Preparation of compounds

### Example 1: Preparation of 1-(4'H,6'H-spiro[cyclopropane-1,7'-thieno[3,2-c]pyran]-4'-yl)-N-methylmethyla mine (Compound 1)

### Synthesis route:

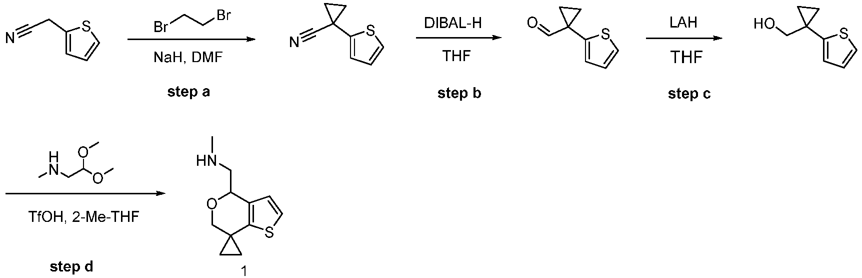

### Step a: synthesis of 1-(thiophen-2-yl) cyclopropanenitrile

Sodium hydride (60%, 1.6g, 40.59mmol) was added to a N, N-dimethylformamide (30mL) solution of 2-(thiophen-2-yl) acetonitrile (2.0g, 16.24mmol)under nitrogen protection and in an ice bath, stirring for 1 hour; then 1,2-dibromoethane (4.0g, 21.3mmol) was slowly added, slowly heating the reaction solution to room temperature and stirring overnight. After the reaction was completed, water (300 mL) was added to quench the reaction in ice bath, extracting with ethyl acetate (300 mL x 3); the extract phase was washed with water (200 mL x 3) and saturated salt solution (200 mL x 3), drying with anhydrous sodium sulfate, filtering and concentrating, then the crude product was separated and purified by column chromatography (petroleum ether/ethyl acetate) to obtain the target product (1.5g, 61.9% yield).

¹H NMR (300 MHz, CDCl₃) δ 7.17 (dd, *J =* 5.1, 0.6 Hz, 1 H), 7.07 - 7.02 (m, 1 H), 6.95 - 6.89 (m, 1 H), 1.78 - 1.67 (m, 2 H), 1.47 - 1.37 (m, 2 H).

### Step b: synthesis of 1-(thiophen-2-yl)cyclopropane aldehyde

Diisobutylaluminum hydride (1M in hexane, 18.8mL, 18.8mmol) was slowly added dropwise to a tetrahydrofuran (100mL) solution of 1-(thiophen-2-yl)cyclopropanenitrile (1.4g, 9.38mmol) in an ice bath and under nitrogen protection, and stirring was continued for 3 hours at room temperature; after the reaction was completed, water (300mL) was added to quench the reaction in ice bath, extracting with ethyl acetate (300 mL x 3); the extract phase was washed with water (300mL x 3) and saturated salt solution (300mL x 3), drying with anhydrous sodium sulfate, filtering and concentrating, then the crude product was separated and purified by column chromatography (petroleum ether/ethyl acetate) to obtain the target product (632.0mg, 44.3% yield).

¹H NMR (300 MHz, CDCl₃) δ 9.34 (s, 1 H), 7.27 - 7.20 (m, 1 H), 7.03 - 6.96 (m, 2 H), 1.73 - 1.64 (m, 2 H), 1.55 - 1.47 (m, 2 H).

### Step c: synthesis of (1-(thiophen-2-yl)cyclopropyl)methanol

A tetrahydrofuran solution of lithium aluminum hydride (2.5M in THF, 3.3mL, 8.30mmol) was slowly added dropwise to a tetrahydrofuran (20mL) solution of 1-(thiophen-2-yl)cyclopropane aldehyde (632.0mg, 4.15mmol) in an ice bath and under nitrogen protection, stirring at room temperature for 2 hours; after the reaction was completed, water (200mL) was added to quench the reaction in ice bath, extracting with ethyl acetate (300mL x 3); the extract phase was washed with water (300mL x 3) and saturated salt solution (300mL x 3), drying with anhydrous sodium sulfate, filtering and concentrating, then the crude product was separated and purified by column chromatography to obtain the target product (570.0mg, 89.0% yield).

¹H NMR (300 MHz, CDCl₃) δ 7.17- 7.11 (m, 1 H), 6.97 - 6.90 (m, 2 H), 3.68 (s, 2 H), 1.96 (s, 1 H), 1.06 - 0.89 (m, 4 H).

### Step d: synthesis of 1-(4'H,6'H-spiro[cyclopropane-1,7'-thieno[3,2-c]pyran] -4'-yl)-N-methylmethylamine

2,2-dimethoxy-N-methylethylamine (386.0mg, 3.24mmol) and trifluoromethanesulfonic acid (0.8mL) were added to a 2-methyltetrahydrofuran (5mL) solution of (1-(thiophen-2-yl)cyclopropyl)methanol (250mg, 1.62mmol), stirring at 80°C for 20 minutes; after the reaction was completed, the pH of reaction solution was adjusted to13 with 15% sodium hydroxide aqueous solution in ice bath, then diluting with water (20mL), extracting with ethyl acetate (20mL x 3); after concentrating the extract phase, the crude product was separated and purified by column chromatography (petroleum ether/ethyl acetate) to obtain the target product (7.3mg, 2.2% yield).

¹H NMR (400 MHz, CD₃OD) δ 7.21 (d, *J =* 5.2 Hz, 1 H), 6.89 (d, *J =* 5.2 Hz, 1 H), 5.11 (d, *J =* 8.8 Hz, 1 H), 3.99 (d, *J* = 11.6 Hz, 1 H), 3.68 (d, *J =* 11.2 Hz, 1 H), 3.60 (dd, *J* = 12.8, 2.4 Hz, 1 H), 3.32-3.25 (m, 1 H), 2.77 (s, 3 H), 1.16 - 0.85 (m, 4 H).

LC-MS [M+H]⁺: 210.1.

### Example 2: preparation of (R)-1-(4'H,6'H-spiro[cyclopropane-1,7'-thieno[3, 2-c]pyran]-4'-yl)-N-methylmethylamine (Compound A)

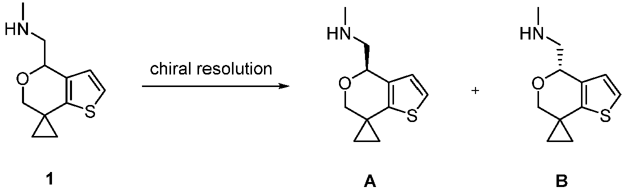

The compound of Example 1 was resolved by chiral chromatographic column to obtain optical enantiomer 1 (Compound A) and optical enantiomer 2 (compound B). Liquid chromatography method: chromatographic column: DAICEL CHIRALPAK IG column; mobile phase A: supercritical CO₂, mobile phase B: methanol (containing 0.1% dimethylamine); detection wavelength: 214nm; flow rate: 1.5 mL/min; column temperature: 35°C; background column pressure: 1800 psi. Mobile phase gradient:

| Time (min) | A(%V/V) | B(%V/V) |
|---|---|---|
| 0.00 | 95 | 5 |
| 0.50 | 95 | 5 |
| 5.50 | 60 | 40 |
| 10.00 | 60 | 40 |

### Optical Enantiomer 1 (Compound A):

RT: 3.62 min; [α]D²⁹ = -59.1 (c 0.163, MeOH); LC-MS [M+H]⁺: 210.1.

¹H NMR (CDCl₃, 600 MHz): δ 7.00 (d, *J* = 5.4 Hz, 1 H), 6.75 (d, *J* = 4.8 Hz, 1 H), 4.93 (dd, *J* = 9.0, 3.0 Hz, 1 H), 3.92 (dd, *J* = 11.4, 1.2 Hz, 1 H), 3.56 (d, *J* = 11.4 Hz, 1 H), 3.02 (dd, *J* = 12.6, 3.0 Hz, 1 H), 2.92 (dd, *J* = 12.6, 9 Hz, 1 H), 2.51 (s, 3 H), 1.05 - 0.90 (m, 4 H).

### Optical Enantiomer 2 (Compound B):

RT: 3.23 min; [α]D²⁹ = 65.1 (c 0.175, MeOH); LC-MS [M+H]⁺: 210.1.

¹H NMR (CDCl₃, 600 MHz): δ 7.00 (d, *J* = 5.4 Hz, 1 H), 6.75 (d, *J* = 4.8 Hz, 1 H), 4.98 (dd, *J* = 9.0, 2.4 Hz, 1 H), 3.92 (d, *J* = 11.4 Hz, 1 H), 3.57 (d, *J* = 11.4 Hz, 1 H), 3.07 (dd, *J* = 12.6, 2.4 Hz, 1 H), 2.95 (dd, *J* = 12.0, 9.0 Hz, 1H), 2.54 (s, 3 H), 1.08 - 0.90 (m, 4 H).

### II. Biological testing of compounds

### Test example 1: test method of TAAR1 receptor cAMP agonist

### 1.1 Experimental materials:

The cAMP detection kit was purchased from Cisbio; the HE3K293 cell line stably expressing TAAR1 receptor was constructed by Shanghai Shujing Biotechnology Co., Ltd.; IBMX was purchased from Sigma; Phenethylamine (PEA); ProxiPlate-384 well plate was purchased from PerkinElmer; HBSS was purchased from Thermo Fisher Scientific Company. PerkinElmer Envision 2105 multifunctional microplate reader, Agilent Bravo liquid workstation, Countstar BioTech cell counter.

### 1.2 Experimental method:

(1). Preparation of experimental buffer: 5× stimulation buffer was diluted to 1× with ddH₂O, adding IBMX with a final concentration of 0.5mM, mixing well for later use.
(2) The frozen cells were thawed quickly in a 37°C water bath, the cell suspension was washed with HBSS buffer, centrifuging at 200xg to remove the frozen storage solution; the pellet was resuspended with an appropriate amount of experimental buffer, taking 20 µL for counting with a cell counter, and diluting to 1.5×10⁶ cells/mL.
(3) 5 µL of cell suspension (1.5×10⁴ cells per well) was added to the ProxiPlate-384 well plate.
(4) The compound to be tested was diluted in the experimental buffer by gradient, and 5 µL was transferred to the reaction plate using Bravo. 5 µL experimental buffer was put in each negative control well, and 5 µL PEA (final concentration 10⁻²M) was put in each positive control well.
(5) Incubating at 37°C for 1 hour.
(6) 10 µL of detection reagent was added to the reaction plate, incubating at room temperature for 1 hour in dark.
(7) Envision 2105 multifunctional microplate reader was used for detection with excitation light 340nm, emission light 620nm and 665nm.The ratio of 665nm/620nm for each test well was calculated.

Activation rate (activity%)=(negative control ratio-compound ratio)/(negative control ratio-positive control ratio)×100%.

The EC₅₀ values of the compounds were calculated using the four-parameter fitting model log(agonist)vs.response--Variable slope(four parameters) in GraphPad Prism.

### 1.3 Experimental results:

The specific results of the compounds of the present application in the TAAR1 receptor agonist activity test through the above scheme are shown in Table 2-1.

**Table 2-1: the results of the agonistic activity of the compounds of the present application on TAAR1 receptors**

| **Compound number** | **TAAR1** | |
|---|---|---|
| | **EC₅₀ (nM)** | **Max Act%** |
| Compound A | 1549 | 95.5% |
| Comparative example 1 | 1646 | 100.5% |
| SEP-363856 | 925 | 108.3% |

### 1.4 Experimental conclusion:

The in vitro experimental results indicate that the compound A of the present application have an agonist effect on the TAAR1 receptors.

### Test example 2: test method of 5-HT_{1A} receptor cAMP agonist

### 2.1 Experimental Materials:

The cAMP detection kit was purchased from Cisbio; the HEK293 cell line stably expressing 5-HT_{1A} receptor was constructed by Shanghai Shujing Biotechnology Co., Ltd.; Serotonin, Forskolin and IBMX were purchased from Sigma; ProxiPlate-384-well plates was purchased from PerkinElmer; HBSS was purchased from Thermo Fisher Scientific. PerkinElmer Envision 2105 multifunctional microplate reader, Tecan D300e picoliter micro-dosing system, Agilent Bravo liquid workstation, Countstar BioTech cell counter.

### 2.2 Experimental methods:

(1) Preparation of experimental buffer: 5×stimulation buffer was diluted to 1× with ddH₂O, adding IBMX with a final concentration of 0.5mM, mixing well for later use.
(2) The cultured cells were digested with trypsin, and the cell suspension was washed with HBSS buffer after the digestion was terminated, centrifuging to remove the culture medium at 200×g; the precipitate was resuspended with an appropriate amount of experimental buffer, taking 20 µL for counting with a cell counter, and diluting to 0.4 × 10⁶ cells/mL.
(3) 5 µL cell suspension (2×10³ cells per well) was added to ProxiPlate-384 well plate.
(4) The compound to be tested was diluted in the experimental buffer by gradient, and 5 µL was transferred to the reaction plate using Bravo. 5 µL experimental buffer was put in each negative control well, and 5 µL Serotonin (final concentration 10⁻⁶M) was put in each positive control well.
(5) Incubating at room temperature for 15 minutes.
(6) Forskolin (final concentration 1.5×10⁻⁷M) was added to the reaction plate using TecanD300e picoliter micro-dosing system.
(7) Incubating at room temperature for 45 minutes.
(8) 10 µL detection reagent was added to the reaction plate, incubating at room temperature for 1 hour in dark.
(9) Envision 2105 multifunctional microplate reader was used for detection with excitation light 340nm, emission light 620nm and 665nm.The ratio of 665nm/620nm for each test well was calculated.

Activation rate (activity%)=(negative control ratio-compound ratio)/(negative control ratio-positive control ratio)×100%

The EC₅₀ values of the compounds were calculated using the four-parameter fitting model log(agonist)vs. response--Variable slope(four parameters) in Graph Pad Prism.

### 2.3 Experimental results:

The specific results of the compounds of the present application in the 5-HT_{1A} receptor agonist activity test through the above scheme are shown in Table 2-2.

**Table 2-2: the results of the agonistic activity of the compounds of the present application on 5-HT_{1A} receptors**

| **Compound number** | 5-HT_{1A} | |
|---|---|---|
| | EC₅₀ (nM) | Max Act% |
| Compound A | 1039 | 95.2% |
| Comparative example 1 | 47746 | 59.5% |
| SEP-363856 | 13130 | 82.4% |

### 2.4 Experimental Conclusion:

The results of in vitro experiments show that the Compound **A** of the present application has agonistic effect on 5-HT_{1A} receptors, suggesting that the compound of the present application can improve negative symptoms and cognitive injury.

### Test example 3: inhibition experiments of the compounds of the present application on high spontaneous activity induced by MK-801 in mice

### 3.1 Experimental materials:

Compounds to be tested: compounds in the examples of the present application, self-made.

(+)-MK-801 bimaleate: purchased from Sigma-Aldrich Company, article number: M107-50MG.

Experimental animals: 18-22g male C57Bl/6J mice, purchased from Shanghai Slack Experimental Animal Co., Ltd.

### 3.2 Experimental method:

**3.2.1 Grouping of animals:** before the start of the experiment, animals were randomly grouped according to body weight.

**3.2.2 Animal adaptation:** before the experiment, the mice were first adapted to the experimental environment for at least 1 hour. That is, the animals were transferred from the breeding room to the laboratory and moved freely in the cage.

### 3.2.3 Administration:

Drug preparation: taking the compound to be tested, adding pure water and performing ultrasonication.

The animals were randomly divided into blank group, model group, and administration group according to body weight, 9 mice /group, and the detailed administration information is shown in the following table:

| Group | Dosage of administration (mg/kg, p.o.) | MK-801 modeling dose(mg/kg, i.p.) | Number of groups | Number of animals (9 mice /group, ♂) |
|---|---|---|---|---|
| Blank group | 0 | 0 | 1 | 9 |
| Model group | 0 | 0.3 | 1 | 9 |
| Compound A | 0.3, 1, 3, 10, 30 | 0.3 | 5 | 45 |
| SEP-363856 | 0.3, 1, 3, 10, 30 | 0.3 | 5 | 45 |

T-30 min oral administration of compound: compound to be tested or solvent (pure water) was administered orally by gavage, and put into test box (test box size length * width * height=27 * 27 * 40cm) immediately after administration, recording the spontaneous activity of the mice within 30 minutes.

T0 min intraperitoneal injection of modeling drug: 30 minutes after compound administration, the mice were taken out, and administered with MK-801 (0.3 mg/kg) by intraperitoneal injection. The blank control group was injected with normal saline. After the administration of MK-801, the animals were immediately put back into the test box, and the test was continued for 150 minutes.

### 3.2.4 Data recording and analysis.

The activities of these animals (movement distance in the test box) were automatically recorded by the camera, and analyzed with ANY MAZE software, and the ED₅₀ values were calculated.

### 3.3 Experimental results: as shown in Table 2-3.

**Table 2-3: Experimental results of the inhibitory effect of the compounds of the present application on high spontaneous activity induced by MK-801 in mice**

| **Group** | **ED₅₀ (mg/kg)** | **MED (mg/kg)** |
|---|---|---|
| Compound A | 4.48 | 10 |
| SEP-363856 | 10.35 | 30 |

| | | |
|---|---|---|
| Note: ED₅₀ is the half effective dose, and MED is the minimum effective dose. | | |

### 3.4 Experimental conclusion:

Through the above scheme, it can be concluded that Compound A of the present application can significantly inhibit the high spontaneous activity induced by MK-801 in mice, and the inhibitory effect is gradually enhanced with the increase of the compound dosage, and there is a good dose-dependent relationship. Compared to SEP-363856, the Compound A of the present application has a lower minimum effective dose and stronger inhibitory effect.

### Test example 4: inhibition experiments of the compounds of the present application on high spontaneous activity induced by PCP in mice

### 4.1 Experimental materials:

Compounds to be tested: compounds of the examples of the present application, self-made.

Phencyclidine hydrochloride (PCP): purchased from Shanghai Yuansi Standard Science Technology Co., Ltd., specification: 5g.

Experimental animals: 18-22g male C57Bl/6J mice, purchased from Shanghai Slack Experimental Animal Co., Ltd.

### 4.2 Experimental method:

**4.2.1 Grouping of animals:** before the start of the experiment, animals were randomly grouped according to body weight.

**4.2.2 Animal adaptation:** before the experiment, the mice were first adapted to the experimental environment for at least 1 hour. That is, animals were transferred from the feeding room to the laboratory, and moved freely in the cage.

### 4.2.3 Administration:

Drug preparation: taking the compound to be tested, adding pure water and performing ultrasonication.

The animals were randomly divided into blank group, model group, and administration group according to body weight, 9 mice /group, and the detailed administration information is shown in the following table:

| Group | Dosage of administration (mg/kg, p.o.) | PCP modeling dose (mg/kg, i.p.) | Number of groups | Number of animals (9 mice /group, ♂) |
|---|---|---|---|---|
| Blank group | 0 | 0 | 1 | 9 |
| Model group | 0 | 5 | 1 | 9 |
| Compound A | 0.1, 0.3, 1, 3, 10 | 5 | 5 | 45 |
| SEP-363856 | 0.1, 0.3, 1, 3, 10 | 5 | 5 | 45 |

T-30 min oral administration of compound: compound to be tested or solvent (pure water) was administered orally by gavage, and put into test box (test box size length * width * height=27 * 27 * 40cm) immediately after administration, recording the spontaneous activity of the mice within 30 minutes.

T0 min intraperitoneal injection of modeling drug: 30 minutes after compound administration, the mice were taken out, and administered with PCP (5 mg/kg) by intraperitoneal injection. The blank control group was injected with pure water. After the administration of PCP, the animals were immediately put back into the test box, and the test was continued for 60 minutes.

### 4.2.4 Data recording and analysis.

The activities of these animals (movement distance in the test box) were automatically recorded by the camera, and analyzed with ANY MAZE software, and the ED₅₀ values were calculated.

### 4.3 Experimental results: as shown in Table 2-4.

**Table 2-4: results of the inhibitory effect of the compounds of the present application on high spontaneous activity induced by PCP in mice**

| **Group** | **ED₅₀ (mg/kg)** | **MED (mg/kg)** |
|---|---|---|
| Compound A | 0.45 | 0.1 |
| SEP-363856 | 1.84 | 3 |

| | | |
|---|---|---|
| Note: ED₅₀ is the half effective dose, and MED is the minimum effective dose. | | |

### 4.4 Experimental conclusion:

Through the above scheme, it can be concluded that the Compound A of the present application can significantly inhibit the high spontaneous activity induced by PCP in mice, and the inhibitory effect is gradually enhanced with the increase of the compound dosage, and there is a good dose-dependent relationship. Compared to SEP-363856, the Compound A of the present application has a lower minimum effective dose and stronger inhibitory effect.

### III. Research on Salt Crystal Forms of the Compound

The free alkali of Compound A is an oily substance. Considering that the oily substance is not conducive to further development into a drug, attempts were made to salify the free form of Compound A, and further study the salt crystal form of compound A.

### Experimental instruments:

| | Model | D2 phaser |
|---|---|---|
| X-ray powder diffraction (XRPD) | Manufactor | Brooke, Germany |
| | radiation source | Copper target |
| | Voltage/Current | 30k V/10 mA |
| | Scanning range | 3° - 60° (2θ value) |
| | Scanning rate | 0.01°/ 0.3s (2θ value) |
| Differential Scanning Calorimeter (DSC) | Instrument model | DSC 3500 |
| | Manufactor | NETZSCH, Germany |
| | Measuring range | 25-300 °C |
| | Heating rate | 10 K/min |
| | Protective gas | N₂ (20 mL/min) |
| | Purge gas | N₂ (20 mL/min) |
| Thermogravimetric analyzer (TGA) | Instrument model | TG 209F3 |
| | Manufactor | NETZSCH, Germany |
| | Measuring range | 30-700 °C |
| | Heating rate | 20 K/min |
| | Protective gas | N₂ (20 mL/min) |
| | Purge gas | N₂ (20 mL/min) |
| High performance | Instrument model | LC-20AD |

| liquid chromatography (HPLC) | Manufactor | Shimadzu Corporation, Japan |
|---|---|---|
| Analytical balance | Instrument model | SQP - One hundred thousandth of a gram balance |
| | Manufactor | Sartorius Scientific Instruments (Beijing) Co., Ltd. |
| Constant temperature heating magnetic stirrer | Instrument model | KMS-151E |
| | Manufactor | KEEZO Technology (Shanghai) Co., Ltd. |
| Blast drying oven | Instrument model | DHG-9055A |
| | Manufactor | Shanghai Yiheng Technology Instrument Co., Ltd. |
| General drug stability test chamber | Instrument model | LHH-150GSD |
| | Manufactor | Shanghai Yiheng Technology Instrument Co., Ltd. |

### 1. Preparation of Salt Crystal Forms of Compound A

### 1.1 Preparation of hydrochloride crystal form I of Compound A

The free alkali Compound A (130.7 mg, 0.63 mmol) prepared in Example 2 and ethyl acetate (2.0 mL) were added to a 25 mL round-bottom flask and dissolved by stirring at room temperature, then 2 M HCl-ethyl acetate solution (0.32 mL, 0.64 mmol) was added, stirring and reacting at room temperature for 3 hours, then filtering with suction, and then drying the solid matter by blast at 50°C for 3 hours to obtain a white solid, which was identified as hydrochloride crystal form I (93.2 mg) of Compound A.

After testing and analysis, it has an XRPD spectrum as shown in Fig.1, a DSC diagram as shown in Fig.2, and a TGA diagram as shown in Fig.3. Combining the results of DSC and TGA, it can be seen that it is a non-solvate.

### 1.2 Preparation of hydrochloride crystal form II of Compound A

Hydrochloride crystal form I (73 mg, 0.3 mmol) of Compound A prepared in section 1.1 above and ethanol (1.0 mL) were added to a 25 mL round-bottom flask, the above suspension was heated to 80°C, stirring and refluxing for 1 hour, then naturally cooling to room temperature, filtering with suction, and then drying the solid matter by blast overnight at 50°C to obtain a white solid, which was identified as hydrochloride crystal form II (20 mg) of Compound A.

After testing and analysis, it has an XRPD spectrum as shown in Fig.4, a DSC diagram as shown in Fig.5, and a TGA diagram as shown in Fig.6. Combining the results of DSC and TGA results, it can be seen that it is a non-solvate.

### 1.3 Preparation of hydrochloride crystal form III of Compound A

The free alkali Compound A (102 mg, 0.49 mmol) prepared in Example 2 and ethyl acetate (2.0 mL) were added to a 25 mL round-bottom flask and dissolved by stirring at room temperature, then 2 M HCl-ethyl acetate solution (0.25 mL, 0.5 mmol) was added, and water (0.25 mL) was added after stirring and reacting for half an hour, continuously stirring at room temperature for 3 days; then filtering with suction, and drying the solid matter at room temperature for 3 hours; the weight was measured to remain unchanged, and a white solid was obtained, which was identified as hydrochloride crystal form III (45 mg) of Compound A.

After testing and analysis, it has an XRPD spectrum as shown in Fig.7, a DSC diagram as shown in Fig.8, and a TGA diagram as shown in Fig.9. Combining the results of DSC and TGA, it can be seen that there is a clear endothermic peak near 84.92°C, corresponding to 6.73% crystal water, i.e., one molecule of water has been removed, indicating that it is a monohydrate.

### 2. Solubility experiment

### 2.1 Experimental purpose:

Investigating the equilibrium solubility of each hydrochloride crystal form of Compound A in water for 24 hours.

### 2.2 Experimental scheme:

Excess samples of hydrochloride crystal form I and hydrochloride crystal form II of Compound A were weighed and placed in different 10 mL centrifuge tubes, and 1 mL of deionized water was added, sealed with a parafilm, shaking at a constant temperature of 37°C and 150 rpm for 24 hours; after passing through a 0.45 µm organic filter, the sample was diluted and injected for HPLC analysis.

**2.2 Experimental results:** the solubility results are shown in Table 3.1 below:

**Table 3.1: Equilibrium solubility of each hydrochloride crystal form of Compound A in water for 24 hours**

| Sample Name | Solubility (mg/mL) |
|---|---|
| Hydrochloride crystal form I | 154.90 |
| Hydrochloride crystal form II | 162.30 |

### 2.4 Experimental Conclusion:

According to the data in the table, it can be seen that the solubility of different crystal forms of Compound A in aqueous medium has significantly increased after salt formation.

### 3. Solution stability experiment

### 3.1 Experimental purpose:

Investigating the solution stability of each hydrochloride crystal form of Compound A.

### 3.2 Experimental scheme and results:

Solutions with pH of 1.0, 3.0, 5.0, and 7.0 solutions were prepared using hydrochloric acid, phosphoric acid, and sodium hydroxide. Appropriate amounts of hydrochloride crystal form I and hydrochloride crystal form II of Compound A were accurately weighed respectively and added to corresponding 20 mL volumetric flasks and labeled, dissolving in media with different pH, passing through a 0.45 µ m organic filters for HPLC analysis. The content changes of the samples from 0-24 hours were monitor, and the results were calculated.

### 3.3 Experimental results:

Hydrochloride crystal form I and hydrochloride crystal form II of Compound A exhibit good stability after 24 hours at different pH conditions (1.0, 3.0, 5.0, and 7.0).

### 4. Solid stability experiment

### 4.1 Experimental purpose:

Investigating the chemical stability of each hydrochloride crystal form of Compound A under high temperature, high humidity, and light conditions.

### 4.2 Experimental scheme:

Appropriate amounts of hydrochloride crystal form I and hydrochloride crystal form II of Compound A were weighed and placed in weighing dishes respectively; three samples for each salt were prepared, and placed respectively in a oven at 60°C, a drying dish with RH 92.5%, and a stability test chamber with an illumination condition of 5000 1x for 85 days. After mixing all samples evenly at day 0, day 10, day 20, and day 85, samples were taken and dissolved in acetonitrile and water, and the content and impurity changes were detected by HPLC-related substance detection method.

**4.3 Experimental results:** the chemical stability results are shown in Table 3.2 below:

**Table 3.2: Chemical stability results of each hydrochloride crystal form of Compound A**

| sample | Experimental conditions | Increase of total impurities (%) | | |
|---|---|---|---|---|
| | | day 10 | day 20 | day 85 |
| Hydrochloride crystal form I of Compound A | High temperature of 60°C | <0.1 | <0.1 | 0.182 |
| | High humidity of RH 92.5% | <0.1 | <0.1 | 0.199 |
| | Illumination of 5000 1x | <0.1 | <0.1 | <0.1 |
| Hydrochloride crystal form II of Compound A | High temperature of 60°C | <0.1 | <0.1 | 0.133 |
| | High humidity of RH 92.5% | <0.1 | <0.1 | 0.145 |
| | Illumination of 5000 1x | <0.1 | <0.1 | <0.1 |

### 4.4 Experimental Conclusion:

The above data indicates that the hydrochloride crystal form I of Compound A and the hydrochloride crystal form II of Compound A of the present application have less increase in impurity content under high temperature, high humidity, or illumination conditions, and have good chemical stability.

### 5. Pharmacokinetic experiments

### 5.1 Experimental purpose:

The mice were administered by oral gavage, and the plasma concentrations of each hydrochloride crystal form of Compound A in mice were determined, the PK parameters were calculated, and the pharmacokinetics were evaluated.

### 5.2 Experimental materials:

(1) Experimental samples: hydrochloride crystal forms I, II, and III of Compound A of the present application, self-made.
(2) Experimental animals: ICR mice, SPF grade, male, Shanghai SLAC Laboratory Animal Co., Ltd.

### 5.3 Experimental scheme:

The compound was administered via oral gavage at a dosage of 5 mg/kg. ICR mice were stratified by body weight and randomly divided into groups of 3 mice each, and fasted overnight before the experiment. Administering according to the prescribed dosage and schedule, and 250 µL of blood was taken from the mandibular vein or saphenous vein of mice by cross sampling at fixed time points to a sample tube containing anticoagulant sodium heparin and placed in wet ice, centrifuging at 4000 r · min⁻¹ for 10 minutes to separate the plasma for LC-MS analysis.

### 5.4 Experimental results and analysis:

The measured blood drug concentration-time data was substituted into the Winnonlin 7.0 program to calculate the main pharmacokinetic parameters. The specific results are shown in Table 3.3.

**Table 3.3: Results of mouse pharmacokinetic experiments**

| Sample | t_{1/2} (h) | Cₘₐₓ (ng/mL) | Tₘₐₓ (h) | AUC_{(inf)} (ng•h/mL) |
|---|---|---|---|---|
| hydrochloride crystal form I of Compound A | 2.8 | 606.3 | 0.5 | 1929.9 |
| hydrochloride crystal form II of Compound A | 2.2 | 488.7 | 0.5 | 1546.6 |
| hydrochloride crystal form III of Compound A | 3.6 | 480.3 | 0.5 | 1452.6 |

### 5.5 Experimental Conclusion:

The results of mouse pharmacokinetic experiments in the table show that different hydrochloride crystal forms of Compound A of the present application can be rapidly absorbed after administration, exhibiting good metabolic properties, with favorable exposure AUC and maximum blood drug concentration Cₘₐₓ.

## Claims

1. An acid salt of a compound (R)-1-(4'H,6'H-spiro[cyclopropane-1,7'-thieno [3,2-c]pyran]-4'-yl)-N-methylmethylamine, wherein the acid salt is a hydrochloride salt.

2. The acid salt according to claim 1, wherein the number of acid molecules in the acid salt is 0.2-3, preferably 0.2, 0.5, 1, 1.5, 2, 2.5 or 3, more preferably 0.5, 1, 2 or 3, and further preferably 1.

3. The acid salt according to any one of claims 1-2, wherein the acid salt is an anhydrate or a hydrate, and when the acid salt is a hydrate, the number of water molecules is 0.2-3, preferably 0.2, 0.5, 1, 1.5, 2, 2.5 or 3, more preferably 0.5, 1, 2 or 3, and further preferably 1.

4. The acid salt according to any one of claims 1-3, wherein the acid salt is in crystal form, preferably hydrochloride crystal form I, hydrochloride crystal form II or hydrochloride crystal form III, wherein:
the X-ray powder diffraction spectrum of the hydrochloride crystal form I comprises diffraction peaks located at 2θ of 12.03 ± 0.2°, 16.08 ± 0.2°, and 26.84 ± 0.2°; preferably comprises diffraction peaks located at 2θ of 12.03 ± 0.2°, 16.08 ± 0.2°, 18.52 ± 0.2°, 25.31 ± 0.2°, and 26.84 ± 0.2°; more preferably comprises diffraction peaks located at 2θ of 12.03 ± 0.2°, 16.08 ± 0.2°, 18.52 ± 0.2°, 20.94 ± 0.2°, 22.06 ± 0.2°, 23.10 ± 0.2°, 24.72 ± 0.2°, 25.31 ± 0.2°, 26.84 ± 0.2°, and 29.60 ± 0.2°; further preferably comprises diffraction peaks located at 2θ of 6.22 ± 0.2°, 12.03 ± 0.2°, 12.36 ± 0.2°, 16.08 ± 0.2°, 18.25 ± 0.2°, 18.52 ± 0.2°, 19.48 ± 0.2°, 20.24 ± 0.2°, 20.94 ± 0.2°, 21.18 ± 0.2°, 22.06 ± 0.2°, 23.10 ± 0.2°, 23.54 ± 0.2°, 24.72 ± 0.2°, 25.31 ± 0.2°, 26.84 ± 0.2°, 27.07 ± 0.2°, 27.77 ± 0.2°, 28.49 ± 0.2°, 29.60 ± 0.2°, 31.05 ± 0.2°, 31.78 ± 0.2°, 32.45 ± 0.2°, 32.79 ± 0.2°, 37.15 ± 0.2°, 39.10 ± 0.2°, 42.73 ± 0.2°, 43.97 ± 0.2°, 44.36 ± 0.2°, and 45.23 ± 0.2°;
the X-ray powder diffraction spectrum of the hydrochloride crystal form II comprises diffraction peaks located at 2θ of 13.36 ± 0.2°, 17.25 ± 0.2°, and 27.74 ± 0.2°; preferably comprises diffraction peaks located at 2θ of 10.30 ± 0.2°, 13.36 ± 0.2°, 17.25 ± 0.2°, 22.77 ± 0.2°, and 27.74 ± 0.2°; more preferably comprises diffraction peaks located at 2θ of 10.30 ± 0.2°, 13.36 ± 0.2°, 15.17 ± 0.2°, 17.25 ± 0.2°, 22.77 ± 0.2°, 24.95 ± 0.2°, 25.28 ± 0.2°, 25.87 ± 0.2°, 27.74 ± 0.2°, and 30.99 ± 0.2°; further preferably comprises diffraction peaks located at 2θ of 8.55 ± 0.2°, 9.96 ± 0.2°, 10.30 ± 0.2°, 11.23 ± 0.2°, 12.49 ± 0.2°, 13.36 ± 0.2°, 15.17 ± 0.2°, 15.99 ± 0.2°, 17.25 ± 0.2°, 18.25 ± 0.2°, 18.83 ± 0.2°, 19.20 ± 0.2°, 20.04 ± 0.2°, 21.11 ± 0.2°, 21.72 ± 0.2°, 22.77 ± 0.2°, 24.13 ± 0.2°, 24.95 ± 0.2°, 25.28 ± 0.2°, 25.87 ± 0.2°, 26.39 ± 0.2°, 27.11 ± 0.2°, 27.74 ± 0.2°, 28.57 ± 0.2°, 29.23 ± 0.2°, 30.99 ± 0.2°, 32.26 ± 0.2°, 34.85 ± 0.2°, 38.72 ± 0.2°, and 40.42 ± 0.2°;
the X-ray powder diffraction spectrum of the hydrochloride crystal form III comprises diffraction peaks located at 2θ of 8.86 ± 0.2°, 17.71 ± 0.2°, and 26.66 ± 0.2°; preferably comprises diffraction peaks located at 2θ of 8.86 ± 0.2°, 13.28 ± 0.2°, 17.71 ± 0.2°, 19.07 ± 0.2°, and 26.66 ± 0.2°; more preferably comprises diffraction peaks located at 2θ of 8.86 ± 0.2°, 13.28 ± 0.2°, 15.75 ± 0.2°, 16.22 ± 0.2°, 17.71 ± 0.2°, 19.07 ± 0.2°, 22.75 ± 0.2°, 23.70 ± 0.2°, 26.66 ± 0.2°, and 31.19 ± 0.2°; further preferably comprises diffraction peaks located at 2θ of 8.86 ± 0.2°, 10.50 ± 0.2°, 12.99 ± 0.2°, 13.28 ± 0.2°, 15.75 ± 0.2°, 16.22 ± 0.2°, 17.71 ± 0.2°, 19.07 ± 0.2°, 20.52 ± 0.2°, 21.01 ± 0.2°, 22.75 ± 0.2°, 23.19 ± 0.2°, 23.70 ± 0.2°, 24.16 ± 0.2°, 24.43 ± 0.2°, 24.91 ± 0.2°, 25.34 ± 0.2°, 25.70 ± 0.2°, 26.09 ± 0.2°, 26.66 ± 0.2°, 27.19 ± 0.2°, 29.06 ± 0.2°, 31.01 ± 0.2°, 31.19 ± 0.2°, 31.41 ± 0.2°, 31.67 ± 0.2°, 32.07 ± 0.2°, 32.98 ± 0.2°, 33.40 ± 0.2°, 36.77 ± 0.2°, 44.77 ± 0.2°, and 49.93 ± 0.2°.

5. The acid salt according to any one of claims 1-4, wherein the acid salt is hydrochloride crystal form I, hydrochloride crystal form II or hydrochloride crystal form III, wherein:
the X-ray powder diffraction spectrum of the hydrochloride crystal form I is basically as shown in Fig.1;
the X-ray powder diffraction spectrum of the hydrochloride crystal form II is basically as shown in Fig.4;
the X-ray powder diffraction spectrum of the hydrochloride crystal form III **is** basically as shown in Fig.7.

6. The acid salt according to any one of claims 1-5, wherein the acid salt is hydrochloride crystal form I, hydrochloride crystal form II or hydrochloride crystal form III, wherein:
the DSC diagram of the hydrochloride crystal form I shows an endothermic peak at 248.07 ± 5°C; preferably the hydrochloride salt form I has a DSC diagram as shown in Fig.2 or has a TGA diagram as shown in Fig.3;
the DSC diagram of the hydrochloride crystal form II shows an endothermic peak at 249.54 ± 5°C; preferably the hydrochloride salt form II has a DSC diagram as shown in Fig.5 or has a TGA diagram as shown in Fig.6;
the DSC diagram of the hydrochloride crystal form III shows endothermic peaks at 84.92 ± 5°C and 245.23 ± 5°C; preferably the hydrochloride salt form III has a DSC diagram as shown in Fig.8 or has a TGA diagram as shown in Fig.9.

7. A method for preparing an acid salt according to any one of claims 1-6, wherein it specifically comprises the following steps:
(1) weighing an appropriate amount of free alkali of the compound (R)-1-(4'H,6'H-spiro[cyclopropane-1,7'-thieno[3,2-c]pyran]-4'-yl)-N-methylmethylamine, and dissolving it with solvent 1;
(2) weighing an appropriate amount of hydrochloric acid, and dissolving it with solvent 2; preferably the amount of the hydrochloric acid is 0.5-2.0 equivalents;
(3) mixing the above two solutions, stirring and reacting at a certain temperature for a certain period of time, filtering with suction, and drying to obtain a target product; or
mixing the above two solutions, stirring and reacting at a certain temperature for a certain period of time, adding solvent 3 to continue stirring and reacting for a certain period of time, filtering with suction, and drying to obtain a target product;
wherein the solvents 1, 2, and 3 are independently selected from the group consisting of: water, methanol, ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, and toluene; wherein solvent 1 and solvent 2 need to be miscible when used.

8. A method for preparing the acid salt according to any one of claims 1-6, wherein it specifically comprises the following steps:
(1) weighing an appropriate amount of the hydrochloride of the compound, and dissolving or suspending it with solvent 4;
(2) refluxing the clarified solution or suspension obtained in step (1) to react at a certain temperature for a certain period of time, cooling to room temperature, filtering with suction, and drying to obtain a target product;
wherein the solvent 4 is selected from the group consisting of: water, anhydrous methanol, anhydrous ethanol, 95% ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, and toluene.

9. A pharmaceutical composition comprising a therapeutically effective amount of the acid salt according to any one of claims 1-6 or a combination thereof, and one or more pharmaceutically acceptable carriers, diluents, or excipients.

10. Use of the acid salt according to any one of claims 1-6, or the pharmaceutical composition according to claim 8 in the preparation of a medicament for preventing and/or treating a neuropsychiatric disease in mammals; preferably the neuropsychiatric disease is central nervous system disease related to 5-hydroxytryptamine receptors, and/or trace amine-associated receptors, and/or dopamine receptors.

11. The use according to claim 10, wherein the neuropsychiatric disease is one or more diseases selected from the group consisting of: schizophrenia, schizophrenia spectrum disease, acute schizophrenia, chronic schizophrenia, NOS schizophrenia, schizoid personality disorder, schizotypal personality disorder, delusional disorder, psychosis, psychotic disorder, brief psychotic disorder, shared psychotic disorder, psychotic disorder due to a physical disease, drug-induced psychosis, psychoaffective disorder, aggression, delirium, Parkinson's psychosis, excitative psychosis, Tourette's syndrome, organic or NOS psychosis, seizure, agitation, post-traumatic stress disorder, behavior disorder, neurodegenerative disease, Alzheimer's disease, Parkinson's disease, dyskinesias, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive nervous disorder, vertigo, pain, neuropathic pain, sensitization accompanying neuropathic pain, inflammatory pain, fibromyalgia, migraine, cognitive impairment, restless leg syndrome, multiple sclerosis, sleep disorder, sleep apnea, narcolepsy, excessive daytime sleepiness, jet lag, drowsy side effect of medications, insomnia, substance abuse or dependence, addiction, eating disorder, sexual dysfunction, hypertension, emesis, Lesche-Nyhane disease, Wilson's disease, autism, and premenstrual dysphoria.
